# EUROPÄISCHE PATENTANMELDUNG

(11) **EP 4 279 141 A1**
(43) Veröffentlichungstag der Anmeldung: **22.11.2023**
(21) Anmeldenummer: 22173482.5
(22) Anmeldetag: 16.05.2022
(51) Int. Cl.: A61Q 11/02, A61K 8/34, A61K 8/44, A61K 8/42, A61K 8/67, A61K 8/55, A61K 8/73, A61K 8/60, A61P 1/02, A61N 5/06

(54) **SPÜLLÖSUNG ZUR PHOTODYNAMISCHEN DESINFEKTION**

(71) Anmelder: Mana Health Technologies GmbH, 8047 Graz (AT)
(72) Erfinder: SMOLLE, Johannes, 8047 Graz (AT); BÖHM, Elisa, 8047 Graz (AT)
(74) Vertreter: Weiser & Voith Patentanwälte Partnerschaft

(57) **Zusammenfassung**

Die vorliegende Erfindung betrifft eine Spüllösung zur photodynamischen Desinfektion, insbesondere Munddesinfektion, enthaltend, jeweils bezogen auf die gesamte Spüllösung, zumindest einen Photosensibilisator aus der Gruppe Riboflavin 5'-phosphat-Natrium in einem Anteil von 0,2 bis 0,6 Gew.-% und ein anderer Photosensibilisator als Riboflavin 5'-phosphat-Natrium in einem Anteil von 0,001 bis 0,05 Gew.-%, zumindest einen Viskositätsmodulator aus der Gruppe Glycerol in einem Anteil von 1,0 bis 5,0 Gew.-% und Hyaluronsäure-Natriumsalz in einem Anteil von 0,01 bis 0,3 Gew.-%, zumindest einen Biofilminhibitor aus der Gruppe Xylitol in einem Anteil von 7,0 bis 25,0 Gew.-%, L-Arginin in einem Anteil von 0,01 bis 0,5 Gew.-% und Urea in einem Anteil von 0,5 bis 3,0 Gew.-%, und Rest Wasserbasis.

## Beschreibung

Die vorliegende Erfindung betrifft eine Spüllösung zur photodynamischen Desinfektion, insbesondere Munddesinfektion.

Bei der photodynamischen Desinfektion werden im Zusammenspiel von Bestrahlung mit Licht und einem Photosensibilisator (auch "Photosensitizer" genannt) schädliche Mikroorganismen, z.B. im Mund oder an einer Schleimhaut, in einer Wunde oder einer natürlichen oder z.B. operativ erzeugten Körperöffnung, an der Hautoberfläche oder einer künstlichen Oberfläche, vernichtet. Bei der Munddesinfektion wird beispielsweise ein meist durch Bakterien gebildeter Biofilm an den Zähnen und am Zahnfleisch beseitigt, wodurch z.B. Karies, Gingivitis und Parodontitis bekämpft werden.

Beim Spülen mit der Spüllösung dringt der Photosensibilisator in die Mikroorganismen ein bzw. legt sich an sie an und wird durch die Bestrahlung aktiviert. Dabei werden freie Radikale, z.B. reaktive Sauerstoffspezies ("ROS"), erzeugt. Die freien Radikale sind weder krebserregend noch für den Menschen genotoxisch und wirken als Biozid, d.h. als eine Substanz, welche insbesondere Krankheitserreger, z.B. Pilze (inklusive deren Sporen), Bakterien, Parasiten und Viren zerstört. Je nach Art des Photosensibilisators wirkt dieser bei der photodynamischen Desinfektion entweder als Katalysator, welcher bei Belichtung die freien Radikale aus der Umgebung erzeugt, z.B. aus einer den Photosensibilisator enthaltenden Lösung; oder der Photosensibilisator gibt bei Bestrahlung selbst freie Radikale ab. Die Wellenlänge der von der Strahlungsquelle abzugebenen Strahlung ist mit dem verwendeten Photosensibilisator und seinem Absorptionsspektrum abzustimmen; sie reicht im Allgemeinen vom Ultraviolett- bis in den Infrarot-Bereich.

In den vergangenen Jahren wurden viele verschiedene Photosensibilisatoren untersucht und zur photodynamischen Desinfektion z.B. von Händen, Mund, Zähnen, Wunden etc. vorgeschlagen; beispielsweise werden in der US 2016/0051833 A1 Chlorophyllin-Natrium-Kupfersalz, Riboflavin, Allurarot, Indigokarmin, Erioglaucin, Tartrazin, Chromotrop, Echtgrün ("Fast Green"), Lissamingrün B, Acridin, Phenazin, Cyanin, Phenothiazin, Porphyrin, Phthalocynin und weitere photodynamisch wirksame Substanzen genannt, welche zur Munddesinfektion einer Zahnpasta oder Mundspülung zugesetzt werden können. Ferner wird darin vorgeschlagen, weitere Inhaltsstoffe beizumengen, z.B. Karies-hemmende, antibakterielle, abrasive, entzündungshemmende, feuchteregulierende Substanzen, Geschmacksstoffe, Farbstoffe usw. usf. Die erzielte Biofilmreduktion liegt bei den vorgeschlagenen Spüllösungen bzw. Zahnpasten mit diesen Photosensibilisatoren nach Bestrahlung über bis zu 15 Minuten zwischen 18% und 72% und damit je nach Photosensibilisator sogar deutlich niedriger oder nur geringfügig höher als bei einer herkömmlichen Mundspülung ohne Photosensibilisator und ohne aufwändige Bestrahlung.

Die Erfindung setzt sich zum Ziel, eine Spüllösung zur photodynamischen Desinfektion, insbesondere Munddesinfektion, zu schaffen, welche wesentlich wirksamer in der Reduktion von Krankheitserregern, insbesondere des Biofilms ist, ohne in der Anwendung schwieriger oder aufwändiger zu sein.

Dieses Ziel wird mit einer Spüllösung zur photodynamischen Desinfektion, insbesondere Munddesinfektion, erzielt, welche zumindest einen Photosensibilisator aus der Gruppe Riboflavin 5'-phosphat-Natrium in einem Anteil von 0,2 bis 0,6 Gew.-% und ein anderer Photosensibilisator als Riboflavin 5'-phosphat-Natrium in einem Anteil von 0,001 bis 0,05 Gew.-%, zumindest einen Viskositätsmodulator aus der Gruppe Glycerol in einem Anteil von 1,0 bis 5,0 Gew.-% und Hyaluronsäure-Natriumsalz in einem Anteil von 0,01 bis 0,3 Gew.-%, zumindest einen Biofilminhibitor aus der Gruppe Xylitol in einem Anteil von 7,0 bis 25,0 Gew.-%, L-Arginin in einem Anteil von 0,01 bis 0,5 Gew.-% und Urea in einem Anteil von 0,5 bis 3,0 Gew.-%, und Rest Wasserbasis enthält. Die angegebenen Anteile sind dabei jeweils auf die gesamte Spüllösung bezogen.

Der vorliegenden Erfindung liegt die Erkenntnis zugrunde, dass nicht alleine die Wahl des Photosensibilisators oder seine Konzentration wesentlichen Einfluss auf die Wirksamkeit der Spüllösung bei Bestrahlung hat, sondern dass gerade das ausgewogene Zusammenspiel der verschiedenen Inhaltsstoffe in entsprechender Konzentration eine überraschende, nicht vorhersehbare Wirkungsverstärkung, nämlich eine Bakterienreduktion um tatsächlich einige Größenordnungen erzeugt. So schwächt der zumindest eine Biofilminhibitor in der angegebenen Konzentration die Integrität des Biofilms, indem der Stoffwechsel der biofilmbildenden Bakterien inhibiert, ihre Verkettung gelöst und/oder die Oberflächenspannung des Biofilms herabgesetzt wird. Der zumindest eine Viskositätsmodulator in der angegebenen Stoffauswahl und Konzentration bewirkt, dass beim Spülen ein dünner, leicht haftender Film mit dem/den Biofilminhibitor/en und Photosensibilisator/en für die nachfolgende Bestrahlung zur photodynamischen Desinfektion auf den zu reduzierenden Bakterienstrukturen gebildet wird, wodurch die Wirkung von Biofilminhibitor/en und Photosensibilisator/en wesentlich verstärkt wird, ohne dass die gesamte Spüllösung beim Bestrahlen z.B. im Mund oder auf der Haut behalten werden muss; d.h. mit der Spüllösung wird einfach und bequem vor dem Bestrahlen z.B. der Mund oder eine andere Körperstelle gespült. Der zumindest eine Photosensibilisator erzeugt letztlich die infolge der Formulierung der Spüllösung besonders starke biozide Wirkung, u.zw. ohne besonders langdauernde oder intensive Bestrahlung, d.h. bequem, einfach und ohne zusätzlichen Aufwand.

Als Photosensibilisator/en kann/können neben dem oder anstelle des genannten Riboflavin 5'-phosphat-Natrium unterschiedliche Substanzen mit photodynamischer Wirkung eingesetzt werden, insbesondere wasserlösliche Photosensibilisatoren, z.B. Phycocyanin, aber auch fettlösliche Photosensibilisatoren, wenn sie z.B. liposomal verkapselt oder mithilfe von Emulgatoren, Nanopartikelträger od.dgl. in der Wasserbasis lösbar gemacht werden. Dabei kann ein einziger Photosensibilisator oder können zwei oder mehr verschiedene Photosensibilisatoren eingesetzt werden.

Die Wasserbasis kann, wie dem Fachmann bekannt ist, z.B. Wasser, destilliertes Wasser oder aromatisiertes Wasser bzw. ein Gemenge aus Wasser und Hydrolat (insbesondere Eukalyptus- oder Pfefferminz-Hydrolat) sein; alternativ ist die Wasserbasis ein wässriges System, dem zur pH-Wert Stabilisierung im Stand der Technik bekannte Puffer beigemengt sind. Beispielsweise zur Verbesserung der Löslichkeit, als Schutz vor Entmischung, zur Konservierung der Spüllösung etc. können der Wasserbasis weitere im Stand der Technik bekannte Inhaltsstoffe beigemengt werden, die jeweils z.B. als Puffersystem, Konservierungsmittel, Trägersystem, Solubilisator, Tensid bzw. Emulgator wirken und/oder Mizellenbildung in der Spüllösung begünstigen.

Vorteilhaft ist, wenn der genannte zumindest eine Photosensibilisator oder einer der (mehreren) Photosenibilisatoren Riboflavin 5'-Phosphat-Natrium in einem Anteil von 0,25 bis 0,45 Gew.-%, bevorzugt in einem Anteil von 0,3 Gew.-%, der Spüllösung ist. Dieser Photosensibilisator hat sich, insbesondere in den genannten Konzentrationsbereichen, in der Spüllösung als besonders wirksam erwiesen. Riboflavin 5'-Phosphat-Natrium ist - neben seinem ausgeprägten photodynamischen Effekt - hautpflegend und fördert als essentielles Vitamin die Haut- auch Schleimhautgesundheit und z.B. die Zahnschmelzfestigkeit.

Alternativ oder ergänzend ist günstig, wenn der oder einer der Photosensibilisator/en Phycocyanin in dem genannten Anteil von 0,001 bis 0,05 Gew.-%, bevorzugt in einem Anteil von 0,005 bis 0,025 Gew.-%, der Spüllösung ist. Ferner ist günstig, wenn der oder einer der Photosensibilisator/en Methylenblau in dem genannten Anteil von 0,001 bis 0,05 Gew.-%, bevorzugt in einem Anteil von 0,005 bis 0,025 Gew.-%, der Spüllösung ist. Diese beiden Photosensibilisatoren, Phycocyanin und Methylenblau, haben sich - jeweils für sich genommen, miteinander oder mit einem anderen Photosensibilisator, insbesondere mit Riboflavin 5'-Phosphat-Natrium, als besonders gut einsetzbar und wirksam erwiesen.

Eine besonders geeignete Spüllösung wird erhalten, wenn der oder einer der Viskosemodulator/en Glycerol in einem Anteil von 2,0 bis 3,0 Gew.-%, bevorzugt in einem Anteil von 2,5 Gew.-%, der Spüllösung ist. Glycerol reguliert nicht nur die Viskosität, sodass in den angegebenen Konzentrationsbereichen der Biofilm effektiv und anhaltend benetzt wird, sondern wirkt überdies schleimhautglättend und befeuchtend. Günstig beeinflusst wird diese Benetzung ferner, wenn der oder einer der Viskosemodulator/en Hyaluronsäure-Natriumsalz in einem Anteil von 0,02 bis 0,1 Gew.-%, bevorzugt in einem Anteil von 0,025 Gew.-%, der Spüllösung ist. Neben seiner viskositätsregulierenden Wirkung ist auch Hyaluronsäure-Natriumsalz ein Feuchthaltemittel und wirkt in den genannten Konzentrationsbereichen antiinflammatorisch.

Bevorzugt ist der oder einer der Biofilminhibitor/en Xylitol in einem Anteil von 15,0 bis 25,0 Gew.-%, bevorzugt in einem Anteil von 15,0 Gew.-%, der Spüllösung. Xylitol ist durch seine bakteriostatische Wirkung in der angegebenen Konzentration ein besonders geeigneter Biofilminhibitor. In einer günstigen Variante der Spüllösung ist der oder einer der Biofilminhibitor/en L-Arginin in einem Anteil von 0,01 bis 0,045 Gew.-%, bevorzugt in einem Anteil von 0,03 Gew.-%, der Spüllösung. L-Arginin wirkt nicht nur antibakteriell sondern auch antistatisch. Vorteilhaft ist ferner, wenn der oder einer der Biofilminhibitor/en Urea in einem Anteil von 1,0 bis 3,0 Gew.-%, bevorzugt in einem Anteil von 1,0 Gew.-%, der Spüllösung ist. Urea greift die Bakterienzellwand an und wirkt ebenfalls antistatisch.

Besonders günstig ist ferner, wenn die Spüllösung zumindest einen Penetrationsverstärker aus der Gruppe Ethanol in einem Anteil von 7,0 bis 20,0 Gew.-%, ein Stoffgemisch aus 1,3-Propanediol und Propanediol in einem gesamten Anteil von 0,5 bis 5,0 Gew.-% und ein Stoffgemisch aus Leuconostoc und Radish Root Ferment Filtrat in einem gesamten Anteil von 0,05 bis 0,15 Gew.-% enthält. Die genannten Anteile sind dabei jeweils auf die gesamte Spüllösung bezogen und ersetzen einen korrespondierenden Anteil der Wasserbasis. Der zumindest eine Penetrationsverstärker verbessert in den genannten jeweils effektiven Konzentrationsbereichen das Eindringen der Spüllösung in die Bakterien oder anderen Mikroorganismen und wirkt allgemein antimikrobiell.

In einer bevorzugten Ausführungsform ist der oder einer der Penetrationsverstärker Ethanol in einem Anteil von 12,0 bis 17,0 Gew.-%, bevorzugt in einem Anteil von 15,0 Gew.-%, der Spüllösung. Ethanol ist als Lösungsmittel nicht nur Penetrationsverstärker sondern wirkt überdies entschäumend, adstringierend, entfettend, reinigend, antibakteriell und reduziert die Viskosität, sodass Ethanol auch mit dem/den Viskosemodulator/en zusammenspielt. Günstig ist dabei, wenn der oder einer der Penetrationsverstärker das Stoffgemisch aus 1,3-Propanediol und Propanediol in einem gesamten Anteil von 1,0 bis 5,0 Gew.-%, bevorzugt in einem gesamten Anteil von 3,0 Gew.-%, der Spüllösung ist. 1,3-Propanediol und Propanediol wirken jeweils einzeln und insbesondere im Stoffgemisch als Penetrationsverstärker an der Bakterienzellmembran. In einer vorteilhaften Variante der Spüllösung ist der oder einer der Penetrationsverstärker das Stoffgemisch aus Leuconostoc und Radish Root Ferment Filtrat in einem gesamten Anteil von 0,05 bis 0,09 Gew.-%, bevorzugt in einem gesamten Anteil 0,08 Gew.-%, der Spüllösung. Dieses Stoffgemisch ist in den genannten Konzentrationsbereichen besonders wirksam antibakteriell und antiseborrhoisch.

Um die Akzeptanz der Spüllösung zu erhöhen, ist ferner günstig, wenn die Spüllösung zumindest einen weiteren Inhaltsstoff aus der Gruppe Pfefferminzextrakt, Ingwerextrakt, Xylitylglucosid, 1,4-Anhydro-D-Xylitol und Glucose enthält, wobei der Anteil jedes dieser weiteren Inhaltsstoffe kleiner als 0,5 Gew.-% der Spüllösung ist. Diese Inhaltsstoffe fördern im Wesentlichen den Geschmack und die Hautverträglichkeit der Spüllösung und unterstützen die Wirkung aller anderen Inhaltsstoffe der Spüllösung. Jeder der weiteren Inhaltsstoffe ersetzt einen korrespondierenden Anteil der Wasserbasis in der Spüllösung.

Die Erfindung wird nachfolgend anhand von Beispielen und den beigeschlossenen Zeichnungen näher erläutert. In den Zeichnungen zeigen:
Fig. 1 das Absorptionsspektrum eines in einer erfindungsgemäßen Spüllösung enthaltenen Photosensibilisators in einem normierten Diagramm über der Wellenlänge;
Fig. 2 die Wirksamkeit des in einer Spüllösung enthaltenen Photosensibilisators von Fig. 1 unter verschiedenen Konzentrationen in einem Balkendiagramm; und
Fig. 3 die Wirksamkeit einer erfindungsgemäßen Spüllösung mit dem Photosensibilisator von Fig. 1 im Vergleich zum Bakterienwachstum mit und ohne Spüllösung bzw. mit und ohne Bestrahlung in einem Balkendiagramm.

Gemäß der vorliegenden Erfindung enthält eine wasserbasierte Spüllösung zur photodynamischen Desinfektion, insbesondere Munddesinfektion, zumindest einen Photosensibilisator, zumindest einen Viskosemodulator zum Einstellen der Viskosität der Spüllösung, zumindest einen Biofilminhibitor und optional zumindest einen Penetrationsverstärker. Der bzw. die Photosensibilisator/en erzielen bei Bestrahlung mit einer Strahlungsquelle nach einem Spülen z.B. des Mundes, einer Schleimhaut, einer Wunde, einer anderen natürlichen oder z.B. operativ erzeugten Körperöffnung, einer Hautoberfläche oder einer künstlichen Oberfläche mit der Spüllösung den photodynamischen Desinfektionseffekt durch Zerstörung von dort anliegenden krankheitserregenden Bakterien, Viren, Pilzen, Parasiten etc. Beispielsweise legen sich im Inneren des Mundes Bakterien als sogenannter Biofilm an die Zähne und das Zahnfleisch an. Der/die Viskosemodulator/en lässt/lassen durch Einstellen der Viskosität der Spüllösung auch nach dem Spülen für die folgende Bestrahlung (z.B. im Mund insbesondere an den Zähnen, dem Zahnfleisch und dem Biofilm) einen dünnen Film der Spüllösung haften. Der/die Biofilminhibitor/en unterstützen ein Eindringen der Spüllösung in den Biofilm oder - allgemein - in Mikroorganismen und Krankheitserreger, was der bzw. die optionale/n Penetrationsverstärker fördern.

Die Wahl und die Konzentrationen der einzelnen Inhaltsstoffe und deren Verhältnis zueinander bestimmen die Wirksamkeit der Spüllösung bei der photodynamischen Munddesinfektion maßgeblich. Für eine außergewöhnlich hohe Wirksamkeit ist der bzw. sind die Photosensibilisator/en aus der Gruppe Riboflavin 5'-phosphat-Natrium in einem Anteil von 0,2 bis 0,6 Gew.-% (Gewichtsprozent) und ein oder mehrere andere Photosensibilisatoren als Riboflavin 5'-phosphat-Natrium jeweils in einem Anteil von 0,001 bis 0,05 Gew.-%, d.h. entweder Riboflavin 5'-phosphat-Natrium im dafür genannten Anteil oder zumindest ein anderer Photosensibilisator jeweils im genannten Anteil für den/die anderen Photosensibilisator/en oder beides. Ferner ist der bzw. sind die Viskositätsmodulator/en aus der Gruppe Glycerol in einem Anteil von 1,0 bis 5,0 Gew.-% und Hyaluronsäure-Natriumsalz in einem Anteil von 0,01 bis 0,3 Gew.-%, ist der bzw. sind die Biofilminhibitor/en aus der Gruppe Xylitol in einem Anteil von 7,0 bis 25,0 Gew.-%, L-Arginin in einem Anteil von 0,01 bis 0,5 Gew.-% und Urea in einem Anteil von 0,5 bis 3,0 Gew.-%. Der Rest der Spüllösung ist eine Wasserbasis, wobei die vorstehend genannten Anteile jeweils auf die gesamte Spüllösung bezogen sind.

Die Wasserbasis kann, wie dem Fachmann bekannt ist, z.B. Wasser, destilliertes Wasser oder aromatisiertes Wasser bzw. ein Gemenge aus Wasser und Hydrolat sein, beispielsweise Eukalyptus-Hydrolat oder Pfefferminz-Hydrolat (letzteres mit der CAS-Registrierungsnummer 84082-70-2 gemäß Chemical Abstract Service, "CAS", der American Chemical Society); alternativ ist die Wasserbasis ein wässriges System, welchem zum Zweck der pH-Wert Stabilisierung (auf einen Wert zwischen 4 und 10, bevorzugt zwischen 5 und 8) im Stand der Technik bekannte Puffersysteme beigemengt sind, beispielsweise Säuren oder Laugen einschließlich ihrer Salze. Dem wässrigen System können optional zur Verbesserung der Löslichkeit, als Schutz vor Entmischung und/oder zur Konservierung der Spüllösung weitere im Stand der Technik bekannte Inhaltsstoffe beigemengt werden, die jeweils als Puffersystem, Konservierungsmittel, Trägersystem, Solubilisator, Tensid bzw. Emulgator wirken und/oder Mizellenbildung in der Spüllösung begünstigen. Überdies kann das wässrige System, falls erforderlich, liposomal verkapselt sein (beispielsweise durch Phospholipide oder Fettsäuren) und/oder eine tensidgeschützte oder tensidfreie Makro-, Mikro- oder Nanoemulsion umfassen, welche beiden letzteren die besonders günstige Eigenschaft auszeichnet, sich in optisch klarem Zustand auszuformen. Der tensidgeschützten oder tensidfreien Makro-, Mikro- oder Nanoemulsion können optional zum Zweck der pH-Wert Stabilisierung, der Verbesserung der Löslichkeit, des Schutzes vor Entmischung und/oder zur Verbesserung der Bioverfügbarkeit der Spüllösung weitere, im Stand der Technik bekannte Inhaltsstoffe beigemengt werden, die jeweils z.B. als Puffersystem, Konservierungsmittel, Trägersystem, Solubilisator, Tensid, Cotensid und/oder Emulgator wirken.

Der bzw. die optionalen Penetrationsverstärker ist/sind aus der Gruppe Ethanol in einem Anteil von 7,0 bis 20,0 Gew.-% und/oder ein Stoffgemisch aus 1,3-Propanediol und Propanediol in einem gesamten Anteil von 0,5 bis 5,0 Gew.-% und/oder ein Stoffgemisch aus Leuconostoc und Radish Root Ferment Filtrat in einem gesamten Anteil von 0,05 bis 0,15 Gew.-%. Die genannten Anteile des/der Penetrationsverstärker sind jeweils auf die gesamte Spüllösung bezogen und ersetzen einen korrespondierenden Anteil der Wasserbasis.

Die Wirkung des zumindest einen Photosensibilisators bei der Desinfektion ist die folgende: Beim Spülen des Mundes oder einer anderen zu desinfizierenden Körperstelle bzw. Oberfläche legt sich der Photosensibilisator an die Mikroorganismen und Krankheitserreger (im Mund: z.B. an den Biofilm) an oder dringt in diese/diesen ein. Durch Bestrahlung wird der Photosensibilisator aktiviert. Dabei werden freie Radikale, z.B. reaktive Sauerstoffspezies ("ROS"), erzeugt, welche als Biozid wirken, d.h. als eine Substanz, welche z.B. Pilze (inklusive deren Sporen), Bakterien, Parasiten und Viren zerstört. Die Wellenlänge der Bestrahlung wird auf den verwendeten Photosensibilisator und sein Absorptionsspektrum abgestimmt; sie reicht im Allgemeinen vom Ultraviolett- bis in den Infrarot-Bereich und kann dabei schmalbandig oder breitbandig sein.

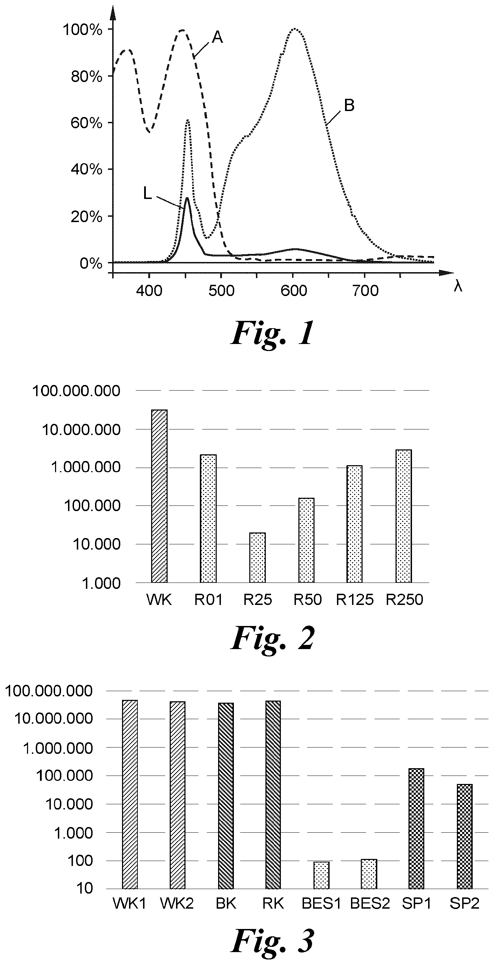

Fig. 1 zeigt die Strahlungsabsorption einer Spüllösung der vorgenannten Art mit einem einzigen Photosensibilisator (hier: Riboflavin 5'-Phosphat-Natrium) bei Bestrahlung durch eine Strahlungsquelle (hier: eine LED) mit einem Breitbandspektrum B (punktierte Linie in Fig. 1), welches einer Farbtemperatur von etwa 3000 Kelvin entspricht. Das Absorptionsspektrum A des hier eingesetzten Photosensibilisators (strichlierte Linie in Fig. 1) hat ein Maximum bei einer Wellenlänge λ von etwa 445 nm. Zur Aktivierung des Photosensibilisators ist daher eine Bestrahlung im Bereich dieser Wellenlänge - oder optional wie im vorliegenden Beispiel mit einer Breitband-Strahlungsquelle, welche diesen Bereich mitumfasst - effektiv. Die durchgezogene Linie L zeigt die tatsächlich absorbierte Strahlung; sie belegt, dass auch bei Wellenlängen deutlich neben dem Absorptionsmaximum des Photosensibilisators (hier z.B. bei 600 nm) noch ein gewisser Teil der Strahlung absorbiert und, wenn auch weniger effizient, mithilfe der Energie der absorbierten Strahlung freie Radikale gebildet werden. Wird mehr als ein Photosensibilisator eingesetzt, ergeben sich im Allgemeinen entsprechend mehrere Absorptionsmaxima, an welche die Strahlungsquelle bzw. ihr Strahlungsspektrum anzupassen ist.

In Fig. 2 ist ausgehend von der vorgenannten Spüllösung mit Riboflavin 5'-Phosphat-Natrium als einzigem Photosensibilisator die - insbesondere photodynamische - Wirkung gegenüber einer Bakteriensuspension der Bakterienart streptococcus mutans ("s. mutans") beispielhaft dargestellt: Der linke Vergleichsbalken WK zeigt in einer Wachstumskontrollmessung die Anzahl der koloniebildenden Einheiten ("colony forming units", CFU) von Mikroorganismen (hier: s. mutans) pro Milliliter (CFU/ml) in der Suspension ohne Behandlung mit Spüllösung und ohne Bestrahlung. Der zugehörige Vergleichswert lag bei ca. 3,2 · 10⁷ CFU/ml. Die weiteren Balken des Diagramms zeigen jeweils die Reduktion des Mikroorganismengehalts bei Verwendung des (hier: einzigen) Photosensibilisators unter verschiedener, auch nichterfindungsgemäßer Konzentration in der Spüllösung nach effektiver Bestrahlung für 300 s mit zwischen 10 und 45 J/cm², im vorliegenden Fall zwischen 17 und 21 J/cm². Bei einem Anteil von 0,01 Gew.-% des Photosensibilisators in der Spüllösung war der Mikroorganismengehalt nach Bestrahlung auf ca. 2,1 · 10⁶ CFU/ml, d.h. um mehr als eine Größenordnung reduziert (Balken R01). Der nächstfolgende Balken R25 zeigt eine Reduktion des Mikroorganismengehalts auf ca. 2,0 · 10⁴ CFU/ml bei einer Photosensibilisator-Konzentration von 0,25 Gew.-% in der Spüllösung, d.h. eine Reduktion um mehr als 3 Größenordnungen bzw. auf weniger als 1/1000 des Vergleichswerts.

Wie die weiteren Balken R50, R125 bzw. R250 zeigen, steigt mit steigender Photosensibilisator-Konzentration von 0,5 Gew.-% (R50), 1,25 Gew.-% (R125) bzw. 2,5 Gew.-% (R250) der Mikroorganismengehalt wieder an, u.zw. auf ca. 1,6 · 10⁵ CFU/ml, ca. 1,1 · 10⁶ CFU/ml bzw. ca. 2,9 · 10⁶ CFU/ml. D.h. trotz höherer Konzentration des Photosensiblisators und infolgedessen zu erwartender höherer Zahl an freien Radikalen sinkt die Wirksamkeit der Desinfektion.

In einer optionalen Formulierung der Spüllösung ist Riboflavin 5'-Phosphat-Natrium in einem Anteil von 0,25 bis 0,45 Gew.-%, insbesondere in einem Anteil von 0,3 Gew.-%, der Spüllösung der einzige Photosensibilisator. Andere Formulierungen der Spüllösung enthalten alternativ oder zusätzlich zu Riboflavin 5'-Phosphat-Natrium einen oder mehrere weitere/n Photosensibilisator/en, insbesondere Phycocyanin und/oder Methylenblau. Der Anteil jedes von Riboflavin 5'-Phosphat-Natrium verschiedenen ("anderen") Photosensibilisators beträgt jeweils 0,001 bis 0,05 Gew.-%, optional 0,005 bis 0,025 Gew.-%, der Spüllösung. Die folgende Tabelle 1 führt Beispiele für geeignete Photosensibilisatoren jeweils mit der diese identifizierenden CAS-Registrierungsnummer an:

**Tabelle 1**

| **Photosensibilisator** | **CAS-Nummer** |
|---|---|
| Riboflavin 5'-Phosphat-Natrium | 130-40-5 |
| | |
| Phycocyanin | 11016-15-2 |
| Methylenblau | 7220-79-3 |
| Toluidinblau 0 | 92-31-9 |
| Azulen | 275-51-4 |
| Thiamin | 67-03-8 |
| Hypericin | 548-04-9 |
| Curcumin | 458-37-7 |
| Carmin | 1390-65-4 |
| Chlorophyll | 479-61-8 |
| Betanin | 7659-95-2 |
| Rose Bengal | 632-69-9 |
| Eosin Y | 17372-87-1 |
| Erythrosin B | 15905-32-5 |
| Indocyaningrün | 3599-32-4 |
| Squaraine | 43134-09-4 |
| Perinaphthenon | 548-39-0 |
| Ruthenium | 7440-18-8 |
| Rhodium | 7440-16-6 |

Die Viskositätsmodulatoren, von welchen zumindest einer in der Spüllösung enthalten ist, und ihre zugehörige CAS-Nummern sind in Tabelle 2 angegeben:

**Tabelle 2**

| **Viskositätsmodulator** | **CAS-Nummer** |
|---|---|
| Glycerol | 56-81-5 |
| Hyaluronsäure-Natriumsalz | 9067-32-7 |

In einer optionalen Ausführungsform werden für die Viskosemodulatoren jeweils engere als die oben angeführten Konzentrationsbereiche vorgegeben: Für Glycerol ein Anteil von 2,0 bis 3,0 Gew.-%, bevorzugt ein Anteil von 2,5 Gew.-%, und/oder für Hyaluronsäure-Natriumsalz ein Anteil von 0,02 bis 0,1 Gew.-%, bevorzugt ein Anteil von 0,025 Gew.-% der Spüllösung.

Die Biofilminhibitoren sind mit ihren jeweiligen CAS-Nummern der folgenden Tabelle 3 zu entnehmen:

**Tabelle 3**

| **Biofilminhibitor** | **CAS-Nummer** |
|---|---|
| Xylitol | 87-99-0 |
| L-Arginin | 74-79-3 |
| Urea | 57-13-6 |

Auch jeder der Biofilminhibitoren kann in einer optionalen Variante jeweils einen engeren, innerhalb der weiter oben angegebenen Konzentrationsbereiche liegenden Konzentrationsbereich haben. So ist Xylitol optional in einem Anteil von 15,0 bis 25,0 Gew.-%, bevorzugt in einem Anteil von 15,0 Gew.-%, L-Arginin optional in einem Anteil von 0,01 bis 0,045 Gew.-%, bevorzugt in einem Anteil von 0,03 Gew.-%, und Urea optional in einem Anteil von 1,0 bis 3,0 Gew.-%, bevorzugt in einem Anteil von 1,0 Gew.-%, in der Spüllösung enthalten.

Tabelle 4 listet die optionalen Penetrationsverstärker zusammen mit den zugehörigen CAS-Nummern:

**Tabelle 4**

| Penetrationsverstärker | **CAS-Nummer** |
|---|---|
| Ethanol | 64-17-5 |
| 1,3-Propanediol/Propanediol | 504-63-2/ 26264-14-2 |
| Leuconostoc/Radish Root Ferment Filtrat | 1686112-10-6/ 84775-94-0 |

In einer optionalen Ausführungsform der Spüllösung haben die Penetrationsverstärker gegenüber ihren weiter oben angegebenen Konzentrationsbereichen jeweils einen engeren Konzentrationsbereich. Ethanol kann dabei beispielsweise in einem Anteil von 12,0 bis 17,0 Gew.-%, bevorzugt in einem Anteil von 15,0 Gew.-%, das Stoffgemisch aus 1,3-Propanediol und Propanediol in einem gesamten Anteil von 1,0 bis 5,0 Gew.-%, bevorzugt in einem gesamten Anteil von 3,0 Gew.-%, und das Stoffgemisch aus Leuconostoc und Radish Root Ferment Filtrat in einem gesamten Anteil von 0,05 bis 0,09 Gew.-%, bevorzugt in einem gesamten Anteil von 0,08 Gew.-%, in der Spüllösung enthalten sein.

Die jeweils zwei Inhaltsstoffe der beiden genannten Stoffgemische 1,3-Propanediol und Propanediol einerseits bzw. Leuconostoc und Radish Root Ferment Filtrat andererseits haben dabei innerhalb des jeweiligen Stoffgemischs beliebige Verhältnisse zueinander; beide Stoffgemische sind jeweils fertig erhältlich, jedoch können sie auch in anderen als den fertig erhältlichen Gemischverhältnissen der Spüllösung zugesetzt werden, insbesondere bis zu 100% des jeweils einen oder anderen der beiden Inhaltsstoffe des jeweiligen Stoffgemischs.

Optional können ferner weitere Inhaltsstoffe in der Spüllösung enthalten sein, welche vorrangig dem Geschmack und der Hautverträglichkeit dienen. Die folgende Tabelle 5 zeigt einige Beispiele für diese Inhaltsstoffe wieder mit der jeweils zugehörigen CAS-Nummer (soweit vorhanden) sowie mit ihren jeweiligen Gewichtsanteilen (in Gew.-%):

**Tabelle 5**

| weiterer Inhaltsstoff | CAS-Nummer | Gewichtsanteil |
|---|---|---|
| Pfefferminzextrakt | 84082-70-2 | < 0,4 |
| Ingwerextrakt | 84696-15-1 | < 0,4 |
| Xylitylglucosid | - | < 0,5 |
| 1,4-Anhydro-D-Xylitol | 53448-53-6 | < 0,34 |
| Glucose | 50-99-7 | < 0,05 |

Der Wasserbasis können optional auch zusätzliche Inhaltsstoffe beigemischt sein, die sich den definierten Gruppen aus Viskositätsmodulatoren, Biofilminhibitoren, Penetrationsverstärkern zuordnen lassen und deren Effektivität jeweils unterstützen, oder solche Inhaltsstoffe, die bereits in herkömmlichen und dem Stand der Technik bekannten Mundspülungen und/oder Zahnpasten eingesetzt werden und zum Zweck des Zahnschmelzschutzes bzw. zur Pflege und Schmerzlinderung der Schleimhaut eingesetzt werden.

Es versteht sich, dass der Anteil der Wasserbasis der Spüllösung um den jeweiligen Gewichtsanteil jedes enthaltenen weiteren (bzw. zusätzlichen) Inhaltsstoffs reduziert ist.

Im Folgenden sind einige Beispiele für die Spüllösung der vorliegenden Offenbarung angegeben.

### Beispiel 1:

Die Spüllösung gemäß Beispiel 1 war wie in der folgenden Tabelle 6 zusammengesetzt (Gewichtsanteile in Gew.-%):

**Tabelle 6**

| Photosensibilisator: | | Gewichtsanteil |
|---|---|---|
| | Riboflavin 5'-Phosphat-Natrium | 0,3 |
| Viskositätsmodulatoren: | | |
| | Glycerol | 2,5 |
| | Hyaluronsäure-Natriumsalz | 0,025 |
| Biofilminhibitoren: | | |
| | Xylitol | 15,0 |
| | L-Arginin | 0,03 |
| | Urea | 1,0 |
| Penetrationsverstärker: | | |
| | Ethanol | 15,0 |
| | 1,3-Propanediol/Propanediol | 3,0 |
| | Leuconostoc/Radish Root Ferment Filtrat | 0,08 |
| | | |
| + Rest Wasserbasis | | 63,065 |

In Fig. 3 ist die Wirkung der Spüllösung gemäß Beispiel 1 anhand zweier Versuchsreihen dargestellt. Die beiden Balken WK1 und WK2 zeigen wieder jeweils Wachstumskontrollmessungen koloniebildender Einheiten der Mikroorganismen in einer Bakteriensuspension (hier: wieder s. mutans): In der ersten Versuchsreihe wurde ein Wert von 4,5 · 10⁷ CFU/ml (WK1), in der zweiten Versuchsreihe ein (ähnlicher) Wert von 4,0 · 10⁷ CFU/ml (WK2) gemessen.

Für die erste Versuchsreihe wurde ferner eine Messung zur Bestrahlungskontrolle nach zweiminütiger Inkubation im Dunkeln und Bestrahlung gemäß Fig. 2 mit einer Strahlungsquelle gemäß Fig. 1 ohne Spüllösung durchgeführt, was gemäß dem Balken BK in Fig. 3 zu einer geringen Reduktion des Messwerts auf 3,7 · 10⁷ CFU/ml führte. Eine weitere Messung (Balken RK) betraf eine 1:1 Vermengung der Bakteriensuspension mit der Spüllösung nach Beispiel 1, was nach siebenminütiger Inkubation und ohne Bestrahlung einen Wert von 4,3 · 10⁷ CFU/ml ergab.

Ferner wurden für die erste Versuchsreihe zwei vergleichende Messungen mit Spüllösung und Bestrahlung durchgeführt, wobei die Inkubationszeit jeweils 120 Sekunden und die Bestrahlungszeit 300 Sekunden betrug. Die Ergebnisse dieser Messungen sind in den Balken BES1 bzw. BES2 gezeigt; die zugehörigen Messwerte betrugen 9,0 · 10¹ CFU/ml bzw. 1,1 · 10² CFU/ml, d.h. rund 5,5 Größenordnungen weniger als der Wert (WK1 bzw. WK2) der Wachstumskontrolle bzw. rund 1/500000.

Für die vergleichende zweite Versuchsreihe wurden zwei verschiedene herkömmliche Mundspülungen (ohne photodynamische Eingenschaften) über die vorgeschriebenen 30 Sekunden dunkel inkubiert; die folgenden Messungen ergaben Werte von 1,8 · 10⁵ CFU/ml (Balken SP1) für die erste Mundspülung bzw. 5,0 · 10⁴ CFU/ml (Balken SP2) für die zweite Mundspülung.

Demnach ist die Spüllösung mit photodynamischem Effekt gemäß Beispiel 1 rund 1000-fach wirksamer als herkömmliche Mundspülungen.

### Beispiel 2:

Gemäß Beispiel 2 bzw. Tabelle 7 enthielt eine alternative Formulierung (Gewichtsanteile in Gew.-%):

**Tabelle 7**

| Photosensibilisator: | | Gewichtsanteil |
|---|---|---|
| | Riboflavin 5'-Phosphat-Natrium | 0,25 |
| Viskositätsmodulatoren: | | |
| | Glycerol | 1,5 |
| | Hyaluronsäure-Natriumsalz | 0,025 |
| Biofilminhibitoren: | | |
| | Xylitol | 8,5 |
| | L-Arginin | 0,015 |
| | Urea | 0,5 |
| Penetrationsverstärker: | | |
| | Ethanol | 15,0 |
| | 1,3-Propanediol/Propanediol | 1,5 |
| | | |
| + Rest Wasserbasis | | 72,71 |

### Beispiel 3:

Gute Wirkung wurde auch mit einer Spüllösung gemäß Beispiel 3 bzw. Tabelle 8 erzielt, welche weitere Inhaltsstoffe im Wesentlichen zur Geschmacksverbesserung enthielt (Gewichtsanteile in Gew.-%):

**Tabelle 8**

| Photosensibilisator: | | Gewichtsanteil |
|---|---|---|
| | Riboflavin 5'-Phosphat-Natrium | 0,3 |
| Viskositätsmodulatoren: | | |
| | Glycerol | 2,5 |
| | Hyaluronsäure-Natriumsalz | 0,025 |
| Biofilminhibitoren: | | |
| | Xylitol | 15,0 |
| | L-Arginin | 0,03 |
| | Urea | 1,0 |
| Penetrationsverstärker: | | |
| | Ethanol | 15,0 |
| | 1,3-Propanediol/Propanediol | 3,0 |
| | Leuconostoc/Radish Root Ferment Filtrat | 0,08 |
| weiterer Inhaltsstoffe: | | |
| | Pfefferminzextrakt | 0,2 |
| | Ingwerextrakt | 0,2 |
| | Xylitylglucosid | 0,3 |
| | 1,4-Anhydro-D-Xylitol | 0,15 |
| | Glucose | 0,04 |
| | | |
| + Rest Wasserbasis | | 62,175 |

### Beispiel 4:

Beispiel 4 enthielt eine vereinfachte Formulierung gemäß der folgenden Tabelle 9 (Gewichtsanteile in Gew.-%):

**Tabelle 9**

| Photosensibilisator: | | Gewichtsanteil |
|---|---|---|
| | Riboflavin 5'-Phosphat-Natrium | 0,25 |
| Viskositätsmodulatoren: | | |
| | Glycerol | 2,7 |
| Biofilminhibitoren: | | |
| | Xylitol | 18,0 |
| | Urea | 2,0 |
| Penetrationsverstärker: | | |
| | Ethanol | 12,5 |
| | 1,3-Propanediol/Propanediol | 4,5 |
| | | |
| + Rest Wasserbasis | | 60,05 |

### Beispiel 5:

Beispiel 5 zeigt in Tabelle 10 eine weitere Formulierung (Gewichtsanteile in Gew.-%):

**Tabelle 10**

| Photosensibilisator: | | Gewichtsanteil |
|---|---|---|
| | Phycocyanin | 0,05 |
| Viskositätsmodulatoren: | | |
| | Glycerol | 3,0 |
| | Hyaluronsäure-Natriumsalz | 0,05 |
| Biofilminhibitoren: | | |
| | Xylitol | 22,4 |
| | Urea | 1,7 |
| Penetrationsverstärker: | | |
| | Ethanol | 16,5 |
| + Rest Wasserbasis | | |
| | | 56,3 |

### Beispiel 6:

Beispiel 6 entsprach einer Formulierung, die in der folgenden Tabelle 11 angeführt ist (Gewichtsanteile in Gew.-%):

**Tabelle 11**

| Photosensibilisator: | | Gewichtsanteil |
|---|---|---|
| | Phycocyanin | 0,01 |
| | Methylenblau | 0,012 |
| Viskositätsmodulatoren: | | |
| | Hyaluronsäure-Natriumsalz | 0,08 |
| Biofilminhibitoren: | | |
| | Xylitol | 12,5 |
| | Urea | 3, 0 |
| Penetrationsverstärker: | | |
| | Ethanol | 13,0 |
| | | |
| + Rest Wasserbasis | | 71,389 |

Es versteht sich, dass die offenbarten Formulierungen und Gewichtsanteile herstellungsbedingten Schwankungen bzw. Messungenauigkeiten unterliegen, deren Größe insbesondere von den eingesetzten Herstellungs- bzw. Messverfahren abhängig ist; so sind je nach Verfahren Abweichungen von den angegebenen Gewichtsanteilen im Bereich von typisch bis zu +/-10% der angegebenen Gewichtsanteile (nicht der gesamten Spüllösung) möglich und mitumfasst.

Die Erfindung ist nicht auf die dargestellten und beschriebenen Ausführungsbeispiele beschränkt, sondern umfasst jene Varianten, Modifikationen und deren Kombinationen, die in den Rahmen der angeschlossenen Ansprüche fallen.

## Patentansprüche

1. Spüllösung zur photodynamischen Desinfektion, insbesondere Munddesinfektion, enthaltend, jeweils bezogen auf die gesamte Spüllösung,
zumindest einen Photosensibilisator aus der Gruppe Riboflavin 5'-phosphat-Natrium in einem Anteil von 0,2 bis 0,6 Gew.-% und ein anderer Photosensibilisator als Riboflavin 5'-phosphat-Natrium in einem Anteil von 0,001 bis 0,05 Gew.-%,
zumindest einen Viskositätsmodulator aus der Gruppe Glycerol in einem Anteil von 1,0 bis 5,0 Gew.-% und Hyaluronsäure-Natriumsalz in einem Anteil von 0,01 bis 0,3 Gew.-%,
zumindest einen Biofilminhibitor aus der Gruppe Xylitol in einem Anteil von 7,0 bis 25,0 Gew.-%, L-Arginin in einem Anteil von 0,01 bis 0,5 Gew.-% und Urea in einem Anteil von 0,5 bis 3,0 Gew.-%,
und Rest Wasserbasis.

2. Spüllösung nach Anspruch 1, wobei der oder einer der Photosensibilisator/en Riboflavin 5'-Phosphat-Natrium in einem Anteil von 0,25 bis 0,45 Gew.-%, bevorzugt in einem Anteil von 0,3 Gew.-%, der Spüllösung ist.

3. Spüllösung nach Anspruch 1 oder 2, wobei der oder einer der Photosensibilisator/en Phycocyanin in dem genannten Anteil von 0,001 bis 0,05 Gew.-%, bevorzugt in einem Anteil von 0,005 bis 0,025 Gew.-%, der Spüllösung ist.

4. Spüllösung nach einem der Ansprüche 1 bis 3, wobei der oder einer der Photosensibilisator/en Methylenblau in dem genannten Anteil von 0,001 bis 0,05 Gew.-%, bevorzugt in einem Anteil von 0,005 bis 0,025 Gew.-%, der Spüllösung ist.

5. Spüllösung nach einem der Ansprüche 1 bis 4, wobei der oder einer der Viskosemodulator/en Glycerol in einem Anteil von 2,0 bis 3,0 Gew.-%, bevorzugt in einem Anteil von 2,5 Gew.-%, der Spüllösung ist.

6. Spüllösung nach einem der Ansprüche 1 bis 5, wobei der oder einer der Viskosemodulator/en Hyaluronsäure-Natriumsalz in einem Anteil von 0,02 bis 0,1 Gew.-%, bevorzugt in einem Anteil von 0,025 Gew.-%, der Spüllösung ist.

7. Spüllösung nach einem der Ansprüche 1 bis 6, wobei der oder einer der Biofilminhibitor/en Xylitol in einem Anteil von 15,0 bis 25,0 Gew.-%, bevorzugt in einem Anteil von 15,0 Gew.-%, der Spüllösung ist.

8. Spüllösung nach einem der Ansprüche 1 bis 7, wobei der oder einer der Biofilminhibitor/en L-Arginin in einem Anteil von 0,01 bis 0,045 Gew.-%, bevorzugt in einem Anteil von 0,03 Gew.-%, der Spüllösung ist.

9. Spüllösung nach einem der Ansprüche 1 bis 8, wobei der oder einer der Biofilminhibitor/en Urea in einem Anteil von 1,0 bis 3,0 Gew.-%, bevorzugt in einem Anteil von 1,0 Gew.-%, der Spüllösung ist.

10. Spüllösung nach einem der Ansprüche 1 bis 9, ferner enthaltend zumindest einen Penetrationsverstärker aus der Gruppe Ethanol in einem Anteil von 7,0 bis 20,0 Gew.-%, ein Stoffgemisch aus 1,3-Propanediol und Propanediol in einem gesamten Anteil von 0,5 bis 5,0 Gew.-% und ein Stoffgemisch aus Leuconostoc und Radish Root Ferment Filtrat in einem gesamten Anteil von 0,05 bis 0,15 Gew.-%, wobei die genannten Anteile jeweils auf die gesamte Spüllösung bezogen sind und einen korrespondierenden Anteil der Wasserbasis ersetzen.

11. Spüllösung nach Anspruch 10, wobei der oder einer der Penetrationsverstärker Ethanol in einem Anteil von 12,0 bis 17,0 Gew.-%, bevorzugt in einem Anteil von 15,0 Gew.-%, der Spüllösung ist.

12. Spüllösung nach Anspruch 10 oder 11, wobei der oder einer der Penetrationsverstärker das Stoffgemisch aus 1,3-Propanediol und Propanediol in einem gesamten Anteil von 1,0 bis 5,0 Gew.-%, bevorzugt in einem gesamten Anteil von 3,0 Gew.-%, der Spüllösung ist.

13. Spüllösung nach einem der Ansprüche 10 bis 12, wobei der oder einer der Penetrationsverstärker das Stoffgemisch aus Leuconostoc und Radish Root Ferment Filtrat in einem gesamten Anteil von 0,05 bis 0,09 Gew.-%, bevorzugt in einem gesamten Anteil von 0,08 Gew.-%, der Spüllösung ist.

14. Spüllösung nach einem der Ansprüche 1 bis 13, ferner enthaltend zumindest einen weiteren Inhaltsstoff aus der Gruppe Pfefferminzextrakt, Ingwerextrakt, Xylitylglucosid, 1,4-Anhydro-D-Xylitol und Glucose, wobei der Anteil jedes dieser weiteren Inhaltsstoffe kleiner als 0,5 Gew.-% der Spüllösung ist und einen korrespondierenden Anteil der Wasserbasis ersetzt.
